# DEMANDE DE BREVET EUROPEEN

(11) **EP 2 228 355 A1**
(43) Date de publication de la demande: **15.09.2010**
(21) Numéro de dépôt: 10290062.8
(22) Date de dépôt: 10.02.2010
(51) Int. Cl.: C07C 4/06, C07C 11/06

(54) **Procédé de conversion directe d'une charge comprenant des oléfines à quatre et/ou cinq atomes de carbone pour la production de propylène**

(30) Priorité: 09.03.2009 FR 0901109
(71) Demandeur: IFP, 92852 Rueil-Malmaison Cédex (FR)
(72) Inventeur: Coupard, Vincent, 38540 Valencin (FR); Digne, Romina, 69007 Lyon (FR)

(57) **Abrégé**

On décrit un procédé de conversion directe d'une charge comprenant des oléfines à quatre et/ou cinq atomes de carbone pour la production de propylène. Il comporte successivement au moins 1) une étape d'oligocraquage des oléfines présentes dans ladite charge conduisant à la production d'au moins un effluent comprenant de l'éthylène, du propylène et des oléfines ayant de 4 à 6 atomes de carbones, 2) une étape de séparation dudit effluent de manière à produire au moins un effluent riche en éthylène, au moins un effluent riche en propylène et au moins un effluent comprenant des oléfines ayant de 4 à 6 atomes de carbone, 3) une étape d'oligomérisation de l'éthylène recueilli à l'issue de ladite étape 2) produisant un oligomérat majoritairement formé de mono-oléfines linéaires en C4 et C6 et 4) le recyclage dudit oligomérat produit à ladite étape 3) à l'entrée de l'unité réalisant la mise en oeuvre de ladite étape d'oligocraquage 1).

## Description

### Domaine de l'invention

L'invention se rapporte au domaine de la production de propylène. Plus précisément, elle concerne un procédé permettant de convertir, au moins en partie en propylène, une charge d'hydrocarbures comprenant des oléfines dont le nombre d'atomes de carbone est majoritairement égal à 4 et/ou 5. Les coupes C4/C5 oléfiniques sont disponibles en quantité importante, souvent excédentaire, dans les raffineries de pétrole et les installations de vapocraquage. Or leur recyclage dans les unités du raffinage est problématique. En effet, le recyclage des coupes oléfiniques C4/C5 au vapocraquage présente des inconvénients car leurs présences altèrent l'efficacité du procédé de vapocraquage et contribuent à la formation de coke en quantité relativement élevée. Par ailleurs, le recyclage des coupes C4/C5 aux unités FCC (craquage catalytique) nécessiterait l'utilisation de conditions beaucoup plus sévères ou de catalyseurs spécifiques, ce qui modifierait de façon profonde le fonctionnement du FCC.

Aussi la présente invention propose une valorisation des coupes oléfiniques C4/C5 en vue de les convertir en propylène.

### Etat de la technique antérieure

Le domaine de la production de propylène a fait l'objet de nombreux travaux ayant donné lieu à de nombreux brevets. En particulier, le brevet FR-B-2 608 595 divulgue un procédé de métathèse qui convertit en propylène un mélange éthylène + n-butène. La demande internationale WO-A-01/04 237 décrit un autre procédé de production de propylène en une étape à partir d'oléfines légères qu'on peut considérer comme une variante du procédé FCC utilisant un catalyseur comprenant une zéolithe ZSM-5. On peut également citer un procédé décrit dans la demande internationale WO-A-99/29 805, dans le brevet EP-B-0 921 181 et la demande de brevet EP-A-0 921 179 : il s'agit d'un procédé utilisant un catalyseur zéolithique de type MFI ayant un rapport Si/Al supérieur ou égal à 180, et de préférence une zéolithe ZSM-5 de rapport Si/Al compris entre 300 et 1000. Ceci à pour effet de limiter les réactions de transfert d'hydrogène responsables de la production de diènes et d'aromatiques. Ce procédé conduit à une faible sélectivité propylène/éthylène.

### Résumé et intérêt de l'invention:

La présente invention a pour objet un procédé de conversion directe d'une charge comprenant des oléfines à quatre et/ou cinq atomes de carbone pour la production de propylène **caractérisé en ce qu**'il comporte successivement au moins :
1) une étape d'oligocraquage des oléfines présentes dans ladite charge conduisant à la production d'au moins un effluent comprenant de l'éthylène, du propylène et des oléfines ayant de 4 à 6 atomes de carbone,
2) une étape de séparation dudit effluent dans au moins une zone de séparation de manière à produire au moins un effluent riche en éthylène, au moins un effluent riche en propylène et au moins un effluent comprenant des oléfines ayant de 4 à 6 atomes de carbone,
3) une étape d'oligomérisation de l'éthylène produit dans l'étape 1) et recueilli à l'issue de ladite étape 2) produisant un oligomérat majoritairement formé de mono-oléfines linéaires en C4 et C6, ladite étape étant mise en oeuvre par catalyse homogène en présence d'un catalyseur comprenant au moins un composé de nickel bivalent, au moins un halogénure d'hydrocarbyl-aluminium et au moins un acide organique de Brönsted,
4) le recyclage dudit oligomérat produit à ladite étape 3) à l'entrée de l'unité réalisant la mise en oeuvre de ladite étape d'oligocraquage 1).

Il a été découvert que, de manière surprenante, un procédé de conversion directe d'une charge comprenant des oléfines à quatre et/ou cinq atomes de carbone pour la production de propylène conduit à un rendement amélioré en propylène et à une amélioration sensible du rapport massique propylène/éthylène par rapport aux procédés antérieurs. L'association des étapes d'oligomérisation de l'éthylène et de recyclage de l'oligomérat formé donne lieu à une sélectivité accrue du procédé de l'invention. Le rapport massique propylène/éthylène obtenu par la mise en oeuvre du procédé de l'invention étant très élevé, il en résulte que ledit procédé selon l'invention présente l'avantage de valoriser exclusivement un seul produit, à savoir le propylène. Le procédé selon l'invention conduisant à une production très faible d'éthylène, il n'est pas utile d'en rechercher un débouché industriel pour le valoriser. Le procédé selon l'invention permet donc une valorisation à moindre coût de l'effluent produit.

### Description détaillée de l'invention :

La présente invention a pour objet un procédé de conversion directe d'une charge comprenant des oléfines à quatre et/ou cinq atomes de carbone pour la production de propylène **caractérisé en ce qu**'il comporte successivement au moins :
1) une étape d'oligocraquage des oléfines présentes dans ladite charge conduisant à la production d'au moins un effluent comprenant de l'éthylène, du propylène et des oléfines ayant de 4 à 6 atomes de carbone,
2) une étape de séparation dudit effluent dans au moins une zone de séparation de manière à produire au moins un effluent riche en éthylène, au moins un effluent riche en propylène et au moins un effluent comprenant des oléfines ayant de 4 à 6 atomes de carbone,
3) une étape d'oligomérisation de l'éthylène produit dans l'étape 1) et recueilli à l'issue de ladite étape 2) produisant un oligomérat majoritairement formé de mono-oléfines linéaires en C4 et C6, ladite étape étant mise en oeuvre par catalyse homogène en présence d'un catalyseur comprenant au moins un composé de nickel bivalent, au moins un halogénure d'hydrocarbyl-aluminium et au moins un acide organique de Brönsted,
4) le recyclage dudit oligomérat produit à ladite étape 3) à l'entrée de l'unité réalisant la mise en oeuvre de ladite étape d'oligocraquage 1).

La charge utilisée pour la mise en oeuvre du procédé de l'invention comprend des oléfines dont le nombre d'atomes de carbone est majoritairement égal à 4 et/ou 5. Ladite charge provient avantageusement d'une unité de vapocraquage ou d'une unité FCC. Toutefois, elle peut également provenir d'une unité de cokéfaction, d'une unité de viscoréduction ou de synthèse Fischer-Tropsch. Plus précisément et de manière préférée, ladite charge est choisie parmi les coupes C4 et C5 de l'effluent du FCC et les coupes C4 et C5 brutes de l'effluent du vapocrquage.

Le terme FCC désigne le procédé de craquage catalytique en lit fluidisé de fractions pétrolières de point d'ébullition supérieur à environ 350 °C, par exemple d'un distillat sous vide, éventuellement de l'huile désasphaltée ou d'un résidu atmosphérique. La coupe C4 de l'effluent du FCC pouvant avantageusement servir de charge pour la mise en oeuvre du procédé selon l'invention comprend principalement de l'isobutène (10 à 25% poids), du butène-1 (10 à 20% poids), du cis-butène-2 (10 à 20% poids), du trans-butène-2 (15 à 25 % poids), de l'isobutane (20 à 30% poids), du n-butane (5 à 20% poids) et du butadiène (< 0,6 % poids). Ladite coupe C4 peut également éventuellement contenir des traces d'hydrocarbures en C3 et C4. La coupe C5 de l'effluent du FCC pouvant avantageusement servir de charge pour la mise en oeuvre du procédé selon l'invention comprend principalement des oléfines dont des dioléfines cycliques (40 à 60% poids), des isoparaffines (30% à 45% poids), des normales paraffines (environ 5% poids) et des cycloparaffines notamment du cyclopentane (1 à 3 % poids). Ladite charge utilisée pour la mise en oeuvre du procédé de l'invention peut être un mélange des coupes C4 et C5 issues du FCC.

Le vapocraquage de naphta (coupe d'hydrocarbures paraffiniques ayant au moins la volatilité dune essence) est un procédé consistant à produire des oléfines par craquage thermique d'hydrocarbures paraffiniques en présence d'eau. Le vapocraquage de naphta produit les grands intermédiaires de la pétrochimie : éthylène, propylène, butadiène et benzène.

La coupe C4 brute de l'effluent du vapocraquage pouvant avantageusement servir de charge pour la mise en oeuvre du procédé selon l'invention comprend principalement du butadiène (40 à 50% poids), de l'isobutène (25 à 30% poids), des n-butènes (20 à 25% poids), des paraffines (5 à 10% poids) et des acétyléniques (0,5 à 3 % poids). La charge utilisée pour la mise en oeuvre du procédé selon l'invention peut également comprendre des fractions d'une essence de vapocraquage (coupe C5), lesdites fractions comprenant typiquement des mono-oléfines (70 à 80% poids), des dioléfines, notamment du cyclopentadiène (10 à 20% poids) et des paraffines (10% poids). Ladite charge utilisée pour la mise en oeuvre du procédé de l'invention peut être un mélange des coupes C4 et C5 brutes issues de l'effluent du vaporcraquage.

Plus généralement, la charge utilisée pour la mise en oeuvre du procédé selon la présente invention est donc une charge comprenant des oléfines à quatre et/ou cinq atomes de carbone, c'est-à-dire une charge oléfinique légère contenant majoritairement, c'est-à-dire plus de 50% poids, de préférence plus de 60% poids, des oléfines en C4 et/ou C5, dont le point final de distillation est généralement inférieur à 320°C, le plus souvent inférieur à 250°C. Souvent, la charge oléfinique utilisée pour la mise en oeuvre du procédé de la présente invention comprend également des composés poly-insaturés, tels que des diènes (dioléfines) notamment à 4 ou 5 atomes de carbone (en particulier du butadiène), des cyclodioléfines dont le cyclopentadiène et des composés acétyléniques pouvant avoir de 2 à 10 atomes de carbone. Ces composés sont des poisons vis à vis des catalyseurs utilisés pour la mise en oeuvre des étapes du procédé de l'invention au sens où ils obstruent les sites acides desdits catalyseurs et entraînent une désactivation par cokage, en particulier du catalyseur utilisé pour la mise en oeuvre de l'étape d'oligocraquage conformément à l'étape 1) du procédé de l'invention. Aussi, pour maximiser la teneur en mono-oléfines présentes dans la charge entrant dans l'unité opérant pour la mise en oeuvre de ladite étape d'oligocraquage, il est avantageux de mettre en oeuvre une étape d'hydrogénation sélective en amont de ladite étape d'oligocraquage de manière à convertir les composés poly-insaturés, en particulier les dioléfines, en mono-oléfines.

Ladite étape d'hydrogénation sélective est facultative mais préférable dès que la charge à traiter comprend une teneur en composés diéniques et acétyléniques supérieure à 100 ppm, de préférence supérieure à 300 ppm et de manière très préférée supérieure à 1000 ppm. Dans le cas où la charge à traiter est une coupe C4 et/ou C5 brute provenant d'une unité de vapocraquage, il est préférable de procéder, en amont de l'étape d'oligocraquage selon l'étape 1) du procédé selon l'invention, à ladite étape d'hydrogénation sélective de manière à convertir les composés diéniques et acétyléniques en mono-oléfines, la coupe C4 brute issue d'une unité de vapocraquage pouvant contenir de 40 % à 50% poids de composés diéniques et acétyléniques, en particulier du butadiène, du vinylacétylène et de l'éthylacétylène et la coupe C5 brute issue d'une unité de vapocraquage pouvant contenir de 10 % à 20 % poids de composés diéniques et acétyléniques, en particulier du cyclopentadiène. Ladite étape d'hydrogénation sélective peut soit traiter la coupe C4 et/ou C5 brute sortant du vapocraqueur soit traiter la coupe C4 après extraction des dioléfines, notamment du butadiène, par solvant, ladite coupe C4, après l'extraction du butadiène par solvant, pouvant contenir de 0,5 à 1% poids de butadiène. Le procédé d'extraction des dioléfines, en particulier du butadiène, par solvant est un procédé connu de l'Homme du métier.

Dans le cas où la charge à traiter est une coupe C4 provenant d'une unité FCC, l'étape d'hydrogénation sélective est optionnelle, la coupe C4 issue d'une unité FCC contenant généralement de 100 à 6000 ppm d'impuretés diéniques et acétyléniques, en particulier du butadiène. Néanmoins, sa mise en oeuvre permet de faciliter la mise en oeuvre des étapes réalisées en aval.

Pour le traitement d'une charge ayant une teneur en dioléfines élevée, c'est-à-dire supérieure à 1 % poids (10000 ppm), l'étape d'hydrogénation sélective est avantageusement mise en oeuvre dans au moins deux réacteurs placés en série, l'effluent de sortie étant au moins en partie préférentiellement recyclé à l'entrée de l'unité d'hydrogénation sélective. Ce recyclage permet d'augmenter la sélectivité de la conversion des dioléfines en mono-oléfines et également de contrôler l'échauffement global du réacteur.

La teneur en dioléfines de l'effluent à l'issue de l'étape d'hydrogénation sélective est comprise entre 10 ppm et 1000 ppm, et préférentiellement entre 50 ppm et 300 ppm. La teneur en acétyléniques de l'effluent à l'issue de l'étape d'hydrogénation sélective est quasiment nulle. Le(s) catalyseur(s) utilisé(s) pour la mise en oeuvre de ladite étape d'hydrogénation sélective est(sont) généralement formé(s) d'au moins un métal du groupe VIII, préférentiellement de nickel ou de palladium, très préférentiellement de palladium, déposé sur au moins un support d'oxyde réfractaire non-acide. En effet, la surface acide externe ne doit pas être trop importante pour limiter les réactions de polymérisation à la surface du catalyseur. Le support préféré du catalyseur d'hydrogénation sélective est constitué d'alumine. Lorsque le palladium est utilisé, sa teneur, sous forme de PdO, est comprise entre 0,1% et 5 % poids, et préférentiellement entre 0,2% et 0,6 % poids. Lorsque le nickel est utilisé, sa teneur, sous forme de NiO, est comprise entre 5% et 25 % poids, préférentiellement entre 7% et 20 % poids.

Les conditions opératoires pour la mise en oeuvre de ladite étape d'hydrogénation sélective sont choisies pour que l'effluent reste à l'état liquide : la température est comprise entre 20°C et 150 °C et la pression totale est comprise entre 0,5 et 4 MPa. La WH, c'est à dire le débit volumique de la charge liquide à 15°C à l'entrée du réacteur d'hydrogénation sélective divisé par le volume de catalyseur dans le réacteur, est avantageusement comprise entre 2 et 8 h⁻¹. L'hydrogène est généralement introduit dans l'unité d'hydrogénation sélective à hauteur de 5 % à 30 % molaire au dessus de la stoechiométrie et préférentiellement de 10 % à 20 % au dessus de la quantité stoechiométrique. Ladite quantité d'hydrogène introduite correspond à la consommation d'hydrogène totale.

Avantageusement, la réaction d'hydrogénation sélective est conduite dans un réacteur à lit fixe généralement à écoulement descendant pour la réaction principale, (c'est le cas quand on a plus de 1 % poids de dioléfines présentes dans la charge à convertir), et avec un catalyseur de préférence constitué de Pd déposé sur alumine, généralement à co-courant ascendant avec l'hydrogène pour la phase de finition de la réaction, de préférence avec un catalyseur constitué de Pd/Ag déposés sur alumine. Cette disposition a pour avantage d'augmenter la sélectivité de l'hydrogénation : hydrogénation des diènes et acétyléniques sans hydrogénation des mono-oléfines.

Conformément au procédé de l'invention, la charge hydrocarbonée comprenant des oléfines à quatre et/ou cinq atomes de carbone ayant éventuellement été préalablement traitée dans une unité d'hydrogénation sélective, est soumise à une étape d'oligocraquage. Selon l'invention, ladite étape d'oligocraquage est opérée en une étape : elle réalise simultanément l'oligomérisation de la charge oléfinique en C4 et/ou C5 pour obtenir une coupe principalement formée d'hydrocarbures en C6 à C15 laquelle subit *in situ* un craquage conduisant à la production de propylène.

La charge entrant dans l'unité mettant en oeuvre l'étape d'oligocraquage présente une concentration en oléfines tel que la pression partielle en oléfines est avantageusement comprise entre 0,03 et 0,09 MPa.

Le catalyseur utilisé dans l'unité pour la mise en oeuvre de ladite étape d'oligocraquage du procédé de l'invention comprend au moins une zéolithe présentant une sélectivité de forme, c'est-à-dire favorisant le craquage en propylène. Ladite zéolithe présente un rapport atomique Si/Al compris entre 50 et 500, et préférentiellement compris entre 75 et 150. Ladite zéolithe est avantageusement choisie parmi les zéolithes présentant un type structural figurant dans la liste suivante : MEL, MFI, NES, EUO, FER, CHA, MFS, MWW. De manière très préférée, elle est choisie parmi les zéolithes de type structural MFI et MEL. En particulier, parmi les zéolithes de type structural MFI, la zéolithe ZSM-5 est préférée et parmi les zéolithes de type structural MEL, la zéolithe ZSM-11 est préférée. Ladite zéolithe à sélectivité de forme peut également être avantageusement choisie parmi les zéolithes NU-85, NU-86, NU-88 et IM-5.

L'utilisation de ces zéolithes à sélectivité de forme présente l'avantage de conduire à une meilleure sélectivité propylène/iso-oléfine, particulièrement une meilleure sélectivité propylène/isobutène, c'est-à-dire à un rapport massique propylène/iso-oléfine, particulièrement un rapport massique propylène/isobutène, élevé dans les effluents de ladite unité d'oligocraquage.

De manière très avantageuse, le catalyseur utilisé dans l'unité pour la mise en oeuvre de ladite étape d'oligocraquage du procédé de l'invention comprend au moins une zéolithe commerciale ZSM-5. Les zéolithes CBV 28014 (rapport atomique Si/Al = 140) et CBV 1502 (rapport atomique Si/Al = 75) commercialisées par la société Zeolyst International et la zéolithe ZSM-5 Pentasil de rapport atomique Si/Al égal à 125 commercialisée par la société Süd-Chemie sont avantageusement utilisées.

De manière préférée, ladite zéolithe à sélectivité de forme est dispersée dans une matrice à base de silice, de zircone, d'alumine ou de silice-alumine, la proportion de zéolithe étant souvent comprise entre 15% et 90 % poids, de préférence entre 20 % et 60 % poids. Ladite étape d'oligocraquage du procédé de l'invention est très préférentiellement mise en oeuvre au moyen d'un catalyseur comprenant au moins une zéolithe présentant une sélectivité de forme et choisie parmi celles décrites ci-dessus et au moins une matrice, en particulier une alumine. Le rapport atomique Si/Al de ladite zéolithe à sélectivité de forme est obtenu soit au moment de la préparation de ladite zéolite soit par un traitement de désalumination ultérieure, la mise en oeuvre d'un tel traitement étant bien connu de l'Homme du métier.

Le catalyseur utilisé dans l'unité pour la mise en oeuvre de ladite étape d'oligocraquage du procédé de l'invention se présente avantageusement sous forme de billes de diamètre généralement compris entre 1,5 mm et 2,5 mm. Ledit catalyseur est généralement mis en oeuvre en lit mobile. Ledit catalyseur peut également être mis en oeuvre en lit fixe, auquel cas un réacteur supplémentaire est utilisé pour ne pas interrompre la production lorsque le catalyseur est régénéré. Dans le cas d'une mise en oeuvre en lit fixe, le ou les réacteurs utilisés travaillent alternativement en réaction puis en régénération selon la technique bien connue dite de "swing" ou de permutation (réacteurs permutables). Indépendamment du mode de mise en oeuvre du réacteur (lit fixe ou lit mobile), la phase de régénération comprend généralement une phase de strippage des composés liquides adsorbés à la surface du catalyseur en présence d'un gaz inerte suivie d'une phase de combustion des dépôts carbonés formés sur le catalyseur, par exemple à l'aide d'un mélange air/azote ou d'air appauvri en oxygène (par exemple par recirculation de fumées de combustion) ou simplement d'air, ladite phase de combustion étant avantageusement suivie d'une phase de calcination. Dans le cas où le catalyseur travaille en lit mobile, la régénération est conduite dans une zone dédiée à cet effet. La régénération peut éventuellement comprendre d'autres phases de traitement et de régénération du catalyseur qui ne seront pas développées ici ne s'agissant pas d'un aspect caractéristique de l'invention.

Conformément à l'invention, l'unité d'oligocraquage mettant en oeuvre ladite étape 1) du procédé de l'invention ne comprend qu'un seul réacteur pour réaliser la réaction, les autres réacteurs éventuellement présents réalisant la régénération du catalyseur présent dans le réacteur fonctionnant en "mode réaction".

L'étape d'oligocraquage selon l'étape 1) du procédé selon l'invention est avantageusement mise en oeuvre dans les conditions opératoires suivantes : une température comprise entre 450°C et 580°C, une vitesse spatiale exprimée en débit horaire de charge à l'entrée du réacteur présent dans l'unité d'oligocraquage par masse de catalyseur (PPH) comprise entre 1 et 60 h⁻¹ préférentiellement entre 22 et 50 h⁻¹, une pression totale comprise entre 0,1 et 0,5 MPa.

Les conditions opératoires pour la mise en oeuvre de la régénération du catalyseur présent dans l'unité d'oligocraquage sont telles que la température est comprise entre 400°C et 650°C et la pression est préférentiellement au moins égale à la pression d'oligocraquage et se situe préférentiellement entre 0,1 et 1 MPa.

L'effluent produit par l'étape d'oligocraquage selon le procédé de l'invention comprend au moins de l'éthylène, du propylène et des oléfines ayant de 4 à 6 atomes de carbone. Il comprend également de l'hydrogène, du méthane ainsi que des hydrocarbures dont le point d'ébullition est supérieur à 75°C (benzène et hydrocarbures ayant au moins 7 atomes de carbone).

Conformément à l'étape 2) du procédé selon l'invention, l'effluent issu de l'étape d'oligocraquage est envoyé dans au moins une zone de séparation de manière à produire séparément au moins un effluent riche en éthylène, au moins un effluent riche en propylène, au moins un effluent comprenant des oléfines ayant de 4 à 6 atomes de carbone, et au moins un effluent servant de coupe essence riche en aromatiques. Ladite zone de séparation opère par distillation et peut comprendre une ou plusieurs colonnes de distillation ; ladite zone de séparation peut également avantageusement opérer par la mise en oeuvre d'étapes d'extraction par solvant en association avec une zone de distillation. L'effluent comprenant les oléfines ayant de 4 à 6 atomes de carbone comprend des oléfines n'ayant pas réagi au cours de l'étape d'oligocraquage. Il est avantageusement recyclé, au moins en partie, à l'entrée de l'unité de mise en oeuvre de ladite étape d'oligocraquage selon le procédé de l'invention. La partie de l'effluent comprenant les oléfines ayant de 4 à 6 atomes de carbone qui n'est éventuellement pas recyclée, peut ensuite être séparée en plusieurs coupes si nécessaire, et selon leur utilisation, en aval du procédé selon l'invention. La coupe essence présente dans l'effluent produit par l'unité d'oligocraquage et recueillie après séparation est une essence riche en aromatiques qui peut être envoyée à un complexe d'extraction d'aromatiques pour, de préférence, être ensuite mélangée au pool essence. Selon un mode préféré de mise en oeuvre de ladite étape 2) du procédé de l'invention, l'effluent comprenant des oléfines ayant de 4 à 6 atomes de carbone est séparé en une coupe comprenant des oléfines en C4 et une coupe comprenant des oléfines en C5 et/ou C6.

Conformément au procédé selon l'invention, la quantité de propylène produite rapportée à la quantité d'oléfines contenues dans la charge entrant dans le réacteur d'oligocraquage est supérieure à 19 %, et de préférence supérieure à 22 % poids.

Conformément à l'étape 3) du procédé de l'invention, l'effluent riche en éthylène recueilli à l'issue de ladite étape de séparation, mise en oeuvre en aval de l'étape d'oligocraquage, est introduit dans une unité mettant en oeuvre une étape d'oligomérisation de manière à convertir l'éthylène en mono-oléfines légères, à savoir principalement en butènes, hexènes, octènes et décènes. L'effluent issu de l'unité mettant en oeuvre ladite étape 3) d'oligomérisation, appelé oligomérat, comprend majoritairement des mono-oléfines linéaires en C4 et C6, à savoir des butènes et hexènes, qui représentent de 60 à 95 % poids des oligomères formés dans ladite unité mettant en oeuvre l'étape d'oligomérisation. Outre la présence majoritaire de butènes et hexènes, l'oligomérat comprend en proportion minoritaire de l'éthylène n'ayant pas réagi ainsi que des mono-oléfines en C8 et C10. L'oligomérat peut également comprendre des traces d'oléfines dont le nombre d'atomes de carbone est supérieur à 10, lesdites traces représentant moins de 5% poids par rapport aux oligomères formés.

De manière générale, l'oligomérisation est l'addition de n oléfines identiques et/ou différentes. L'oligomérisation se distingue de la polymérisation par une addition de molécules en nombre limité, le chiffre n étant, selon l'invention, pour la plus grande partie pondérale au moins des oligomères, compris entre 2 et 10, et préférentiellement entre 2 et 6.

Conformément à l'étape 3) du procédé de l'invention, l'oligomérisation de l'éthylène est mise en oeuvre par catalyse homogène, c'est-à-dire en présence d'un catalyseur soluble dans l'éthylène et ses produits d'oligomérisation pour former une seule phase liquide.

Pour une mise en oeuvre de l'étape 3) d'oligomérisation de l'éthylène par catalyse homogène, l'Homme du métier pourra utilement se référer à l'enseignement des brevets US 7.235.703 et US 4.362.650. Le catalyseur présent dans l'unité mettant en oeuvre ladite étape d'oligomérisation comprend au moins un composé de nickel bivalent, au moins un halogénure d'hydrocarbyl-aluminium et au moins un acide organique de Brönsted. De manière facultative, ledit catalyseur peut également contenir au moins un anhydride d'acide carboxylique.

Les composés de nickel bivalent sont de préférence des carboxylates de nickel de formule générale (RCOO)₂Ni où R est un radical hydrocarbyle, par exemple alkyle, cycloalkyle, alkényle, aryle, aralkyle ou alkaryle. Des exemples de composés de nickel bivalent sont les sels de nickel bivalent suivants : octoate, éthyl-2-hexanoate, décanoate, stéarate, oléate, salicylate et hydroxydécanoate.

Les halogénures d'hydrocarbyl-aluminium sont de préférence des dihalogénures d'hydrocarbyl-aluminium répondant à la formule AlRX₂, dans laquelle R est un radical hydrocarbyle et X un halogène (F, Cl, Br, I). Il s'agit par exemple du dichloroéthylaluminium, du dichloroisobutylaluminium et du dibromoéthylaluminium. Ces dihalogénures d'hydrocarbylaluminium peuvent être avantageusement enrichis avec des trihalogénures d'aluminium (AlX₃) comme par exemple le trichlorure d'aluminium.

Les acides organiques de Brönsted ont de préférence un pKa à 20°C au maximum égal à 3 et sont choisis de préférence dans le groupe formé par les acides halogénocarboxyliques de formule RCOOH dans laquelle R est un radical alkyle halogéné. Des exemples d'acides organiques de Brönsted sont l'acide trifluoroacétique, l'acide trichloroacétique et l'acide tribromoacétique.

Les anhydrides d'acide carboxylique sont des composés de formule (RCO)₂O dans laquelle R est un radical hydrocarbyle pouvant contenir de manière facultative un ou plusieurs atomes d'halogène. On peut citer à titre d'exemples les anhydrides octoïque, éthyl-2-hexanoïque, décanoïque, stéarique, oléique, trifluoroacétique, monofluoroacétique, trichloroacétique, monochloroacétique, pentafluoropropionique ou heptafluorobutyrique.

Les différents composés constituant le catalyseur peuvent être mélangés dans un ordre quelconque. Cependant, il est préférable de mélanger d'abord le composé de nickel avec l'acide organique de Brönsted, puis d'introduire ensuite le composé d'aluminium.

Un préconditionnement du catalyseur peut être effectué avant la mise en contact du catalyseur avec l'éthylène, c'est-à-dire que les constituants du catalyseur peuvent être mélangés au préalable dans un solvant hydrocarboné, comme par exemple les produits de l'oligomérisation de l'éthylène. Ce préconditionnement du catalyseur permet d'augmenter l'activité du catalyseur dans l'oligomérisation de l'éthylène.

Le catalyseur présent dans ladite unité opérant l'étape d'oligomérisation se présente sous forme liquide. En fonction de la composition chimique dudit catalyseur, les proportions pondérales de chacun des composants du catalyseur sont à contrôler lors de la synthèse du catalyseur. Le rapport molaire de l'halogénure d'hydrocarbylaluminium au composé de nickel, exprimé par le rapport Al/Ni, est de 2/1 à 50/1, et de préférence de 2/1 à 20/1.

Le rapport molaire de l'acide de Bronsted au composé de nickel est de 0,25/1 à 10/1, et de préférence de 0,25/1 à 5/1. Si le catalyseur comprend de l'anhydride d'acide carboxylique, le rapport molaire de l'anhydride d'acide carboxylique au composé de nickel est avantageusement compris entre 0,001/1 et 1/1, très avantageusement entre 0,01/1 et 0,5/1.

Conformément au procédé de l'invention, l'étape d'oligomérisation mise en oeuvre par catalyse homogène est réalisée en continu : la solution catalytique est injectée dans l'unité opérant l'étape d'oligomérisation en même temps que l'éthylène. L'unité opérant ladite étape d'oligomérisation de l'éthylène par catalyse homogène comprend un ou plusieurs réacteurs de type parfaitement agité, en série, avec recycle d'une partie, au moins, de l'effluent du réacteur dans le réacteur, ce recycle ayant été avantageusement refroidi. Le recycle a un rôle de diluant thermique car la réaction est exothermique et d'extracteur de chaleur. Le recycle est interne à l'unité d'oligomérisation. Dans le cas où l'unité d'oligomérisation comprend plusieurs réacteurs, la charge et le système catalytique sont introduits en continu dans un premier réacteur, le système catalytique étant également avantageusement introduit dans l'un quelconque des réacteurs secondaires.

Les conditions opératoires dans le(s) réacteur(s) opérant l'étape d'oligomérisation par catalyse homogène sont telles que la température est comprise entre -20 °C et +60 °C et la pression suffisante pour permettre l'existence d'une phase liquide dans le(s) réacteur(s). De manière préférée, la pression totale dans le(s) réacteur(s) se situe entre 3 et 8 MPa.

A la sortie du dernier réacteur de l'unité d'oligomérisation, une partie du catalyseur se trouve en mélange avec l'oligomérat et l'éthylène qui n'a pas réagi. Pour éliminer le catalyseur des hydrocarbures, on ajoute une solution aqueuse qui va permettre le transfert du catalyseur de la phase hydrocarbonée à la phase aqueuse. Par séparation de la phase aqueuse et de la phase hydrocarbonée, non miscibles, le catalyseur est éliminé des hydrocarbures. L'élimination du catalyseur de l'effluent du réacteur d'oligomérisation peut se faire par exemple à l'aide d'ammoniac et/ou d'une solution aqueuse de soude et/ou d'une solution aqueuse d'acide sulfurique.

L'éthylène non converti au cours de l'étape d'oligomérisation et les éventuels composés inertes comme l'éthane ou le méthane, présents dans la coupe riche en éthylène issue de l'étape 2) de séparation du procédé de l'invention, sont séparés des oligomères par distillation.

Conformément à l'étape 4) du procédé de conversion selon l'invention, l'oligomérat produit lors de ladite étape 3) est recyclé à l'entrée de l'unité réalisant la mise en oeuvre de ladite étape d'oligocraquage 1). De manière avantageuse, l'effluent issu de ladite étape d'oligomérisation 3) est envoyé dans une zone de séparation permettant de recueillir séparément au moins un effluent riche en éthylène n'ayant pas été converti et au moins un effluent comprenant les oligomérats formés au cours de ladite étape d'oligomérisation 3), ledit effluent comprenant lesdits oligomérats étant seul recyclé à l'entrée de l'unité réalisant la mise en oeuvre de ladite étape d'oligocraquage 1).

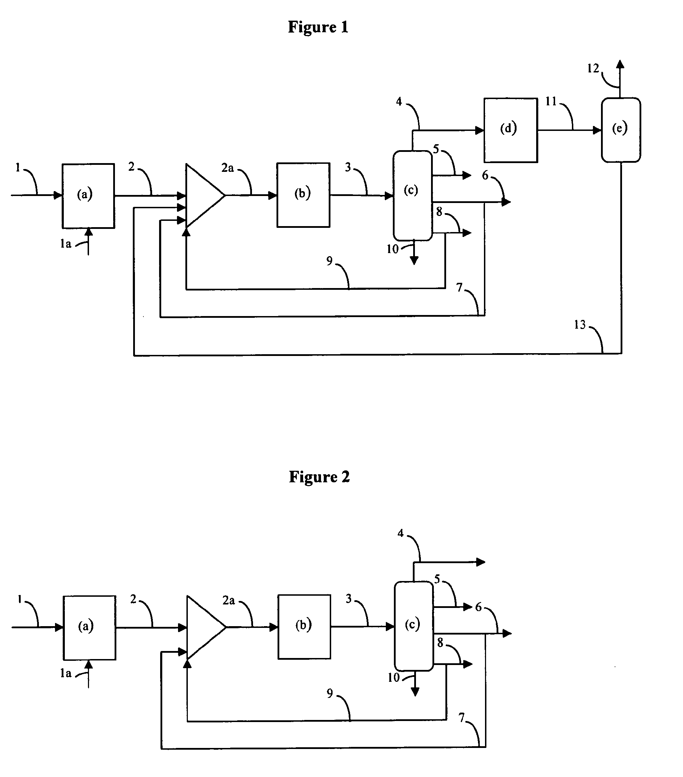

On donne ci-après une description du procédé de conversion de l'invention tel qu'il est représenté sur la figure 1. Cette représentation concerne le cas où la charge à traiter comprend des impuretés diéniques et acétyléniques qui nécessitent d'être éliminées au cours d'une étape d'hydrogénation sélective.

La charge à traiter est introduite par le flux (1) dans une unité (a) mettant en oeuvre l'étape d'hydrogénation sélective laquelle conduit à la production d'un effluent (2) qui est mélangé avec les flux de recycle (7), (9) et (13) pour former le flux d'alimentation (2a) de l'unité (b) mettant en oeuvre l'étape d'oligocraquage. Le flux (1a) correspond à l'hydrogène introduit et totalement consommé dans l'étape d'hydrogénation sélective. L'effluent (3) sortant de ladite unité d'oligocraquage est envoyé dans une zone de séparation (c) de manière à recueillir un effluent (4) constitué d'une coupe légère riche en éthylène, un effluent (5) riche en propylène, un effluent (6) contenant majoritairement des hydrocarbures C4 dont une partie est recyclée par le flux (7) à l'entrée de l'unité (b) d'oligocraquage, un effluent (8) contenant majoritairement des hydrocarbures C5 ou C5/C6 dont une partie est recyclée par le flux (9) à l'entrée de l'unité (b) d'oligocraquage et un effluent (10) comprenant des composés aromatiques et oléfiniques dont les points d'ébullition se situent dans l'intervalle des essences, soit généralement de 60°C à 250 °C.

La coupe légère riche en éthylène (4) est envoyée dans une unité (d) mettant en oeuvre l'étape d'oligomérisation d'éthylène. L'effluent (11) sortant de ladite unité (d) est séparé dans une colonne à distiller (e) en deux coupes, à savoir une coupe hydrocarbonée (12) majoritairement constituée de fractions en C2 et de composés plus légers obtenue en tête de colonne et un oligomérat (13) majoritairement constitué d'oléfines en C4, C6 et C8 obtenu en fond de colonne, ledit oligomérat étant recyclé vers l'unité (b) mettant en oeuvre l'étape d'oligocraquage.

### Exemple 1 (comparatif) : procédé de conversion directe d'une charge comprenant des oléfines en C4 pour la production de propylène.

Cet exemple est illustré par la figure 2. Les unités (a), (b) et (c) ainsi que les flux 1 à 10 ont la même signification que celle donnée pour la description de la figure 1. La charge à traiter (1) dans le procédé est une coupe C4 brute de vapocraquage dont la composition est donnée dans le tableau 1. Elle est introduite dans l'unité d'hydrogénation sélective où est également introduit de l'hydrogène (1a). L'unité (a) mettant en oeuvre l'étape d'hydrogénation sélective fait appel à l'emploi de deux réacteurs (non représenté sur la figure). Le premier réacteur utilise un catalyseur Pd / Al₂O₃ à 0,3 % poids de Pd sur une alumine de 69 m²/g de surface spécifique (LD 265, Axens). Il fonctionne à 50°C de manière adiabatique en lit fixe descendant, à 3 MPa. Pour que la réaction reste en phase liquide, un recyclage (provenant de l'effluent de l'hydrogénation sélective) égal à 20 fois le débit massique de charge est utilisé. Le rapport global H₂/butadiène total est de 1,05 en mole / mole à l'entrée du réacteur soit une quantité d'hydrogène égale à 5% molaire au-dessus de la stoechiométrie. Le second réacteur dit "réacteur finisseur" est un réacteur à écoulement ascendant, utilisant un catalyseur Pd + Ag déposé sur alumine, soit 0,2 % poids de Pd et 0,1 % poids d'Ag déposés sur une alumine de 69 m²/g de surface BET (LD 271, Axens). La température est réglée à 35 °C, la pression à 2,6 MPa. La quantité totale de catalyseur utilisée pour la réaction d'hydrogénation sélective est de 3,5 m³ correspondant à 5 m³ de charge fraîche traitée par heure par m³ de catalyseur, soit une VVH égale à 5h⁻¹.

L'unité (b) mettant en oeuvre l'étape d'oligocraquage comprend un réacteur fonctionnant à 0,28 MPa, à 545°C, avec une PPH (débit massique de charge à l'entrée du réacteur par masse de catalyseur) de 25 h⁻¹ par rapport à la charge entrant dans le réacteur réalisant la réaction d'oligocraquage. Un seul réacteur adiabatique à écoulement descendant, mis en oeuvre en lit mobile, en phase gaz est utilisé. Le temps de cycle entre deux régénérations successives est de 48 h. La régénération du catalyseur est réalisée en trois étapes : une phase de strippage à l'azote pendant 6 heures, une phase de combustion avec un mélange azote et air (azote : 80%, air : 20%) pendant 12 heures et avec une montée en température de 400 à 600°C, et pour terminer une phase de calcination à 600°C pendant 2 heures avec de l'air.

Le catalyseur utilisé est composé de 30% poids de zéolithe ZSM-5 (zéolithe CBV 28014, Zeolyst International) de rapport atomique Si/Al de 140 et de 70% poids d'alumine gamma. Il est préparé sous forme de billes de 1,8 mm de diamètre mis en forme par la technique de l'"oil drop" (coagulation en gouttes). Le ratio massique propylène/isobutène dans l'effluent de l'étape d'oligocraquage est égal à 1,5. Le ratio massique propylène/(isobutène + iso-oléfines en C5) dans l'effluent de l'étape d'oligocraquage est égal à 1,08.

Les coupes C4 et C5/C6 issues de l'unité (b) d'oligocraquage et recueillies à la suite de l'étape de séparation (c) sont recyclées à hauteur de 80% poids à l'entrée de ladite unité (b) via le flux (7) pour la coupe C4 et le flux (9) pour la coupe C5/C6.

La composition et le débit total des différents flux figurant sur la figure 2 sont indiqués dans le tableau 1.

Le rendement global de la coupe C3 est de 37,6 % poids par rapport à la charge fraîche ((1)+(1a)) du procédé global. Cette coupe C3 contient 93% poids de propylène. Le ratio massique propylène/éthylène sortant du procédé est égal à 4,3.

**Tableau 1 : Bilan matière (débits en kg/h)**

| | | 1 | 1a | 2 | 2a | 3 | 4 | 5 | 6 | 7 | 8 | 9 | 10 |
|---|---|---|---|---|---|---|---|---|---|---|---|---|---|
| | H2 + methane + ethane | | 155 | | | 80 | 80 | | | | | | |
| | Ethylène | | | | | 823 | 823 | | | | | | |
| C3 | Propylène + propane | | | | | 3823 | | 3823 | | | | | |
| C4 | n oléfines | 2340 | | 6256 | 9899 | 4555 | | | 911 | 3644 | | | |
| | iso oléfines | 2889 | | 2889 | 4763 | 2342 | | | 468 | 1873 | | | |
| | dioléfines | 3976 | | 2 | 9 | 9 | | | 2 | 7 | | | |
| | paraffines | 795 | | 1008 | 7126 | 7647 | | | 1529 | 6118 | | | |
| C5 | n + iso oléfines | | | | 1129 | 1412 | | | | | 282 | 1129 | |
| | cyclooléfines | | | | 28 | 35 | | | | | 7 | 28 | |
| | dioléfines | | | | 0 | 0 | | | | | 0 | 0 | |
| | paraffines | | | | 638 | 798 | | | | | 160 | 638 | |
| C6 | oléfines | | | | 183 | 229 | | | | | 46 | 183 | |
| | paraffines | | | | 314 | 393 | | | | | 79 | 314 | |
| | Benzène + C7⁺ | | | | 0 | 1921 | | | | | | | 1921 |
| | Coke | | | | | 24 | | | | | | | |
| | TOTAL | 10000 | 155 | 10155 | 24090 | 24090 | 903 | 3823 | 2910 | 11642 | 573 | 2294 | 1921 |

### Exemple 2 (comparatif) : procédé de conversion directe d'une charge comprenant des oléfines en C4 pour la production de propylène.

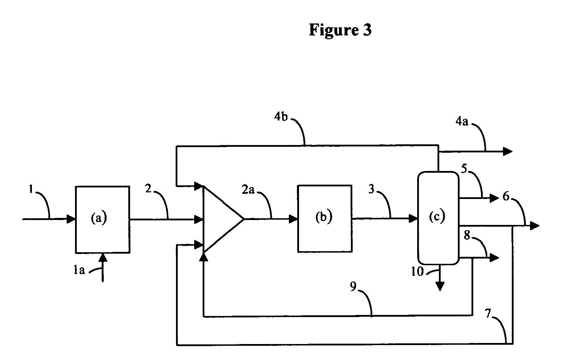

Cet exemple est illustré par la figure 3. La charge à traiter (1) dans le procédé est une coupe C4 brute de vapocraquage identique à celle de l'exemple 1. Les unités (a), (b) et (c) ainsi que les flux 1 à 3 et 5 à 10 ont la même signification que celle donnée pour la description de la figure 1.

L'unité (a) mettant en oeuvre l'étape d'hydrogénation sélective est identique à celle de l'exemple 1. Elle fait appel à l'emploi des 2 réacteurs décrits dans l'exemple 1.

L'unité (b) mettant en oeuvre l'étape d'oligocraquage opère dans les mêmes conditions que celles de l'exemple 1 mais l'effluent riche en éthylène recueilli à l'issue de la zone de séparation (c) est recyclé à hauteur de 80% poids à l'entrée de l'unité (b) d'oligocraquage via le flux (4b). Une purge (4a) de l'effluent riche en éthylène est effectuée.

Le ratio massique propylène/isobutène dans l'effluent de l'étape d'oligocraquage est égal à 1,5. Le ratio massique propylène/(isobutène + iso-oléfines en C5) dans l'effluent de l'étape d'oligocraquage est égal à 1,08.

La composition et le débit total des différents flux figurant sur la figure 3 sont indiqués dans le tableau 2.

Le rendement global de la coupe C3 est de 40,0 % poids par rapport à la charge fraîche ((1) + (1a)). Cette coupe C3 contient 93% poids de propylène. L'augmentation du rendement en propylène est donc de 2,4% par rapport à l'exemple 1. Le ratio massique propylène/éthylène des flux sortant du procédé est alors égal à 13.

**Tableau 2 : Bilan matière (débits en kg/h)**

| | | 1 | 1a | 2 | 2a | 3 | 4a | 4b | 5 | 6 | 7 | 8 | 9 | 10 |
|---|---|---|---|---|---|---|---|---|---|---|---|---|---|---|
| | H2 + methane + ethane | | 155 | | 68 | 427 | 85 | 342 | | | | | | |
| | Ethylène | | | | 233 | 1459 | 292 | 1167 | | | | | | |
| C3 | Propylène + propane | | | | 0 | 4067 | | | 4067 | | | | | |
| C4 | n oléfines | 2340 | | 6256 | 10132 | 4845 | | | | 969 | 3876 | | | |
| | iso oléfines | 2889 | | 2889 | 4882 | 2491 | | | | 498 | 1993 | | | |
| | dioléfines | 3976 | | 2 | 9 | 9 | | | | 2 | 7 | | | |
| | paraffines | 795 | | 1008 | 7259 | 7813 | | | | 1563 | 6251 | | | |
| C5 | n + iso oléfines | | | | 1201 | 1502 | | | | | | 300 | 1201 | |
| | cyclooléfines | | | | 30 | 37 | | | | | | 7 | 30 | |
| | dioléfines | | | | 0 | 0 | | | | | | 0 | 0 | |
| | paraffines | | | | 679 | 849 | | | | | | 170 | 679 | |
| C6 | oléfines | | | | 195 | 244 | | | | | | 49 | 195 | |
| | paraffines | | | | 334 | 418 | | | | | | 84 | 334 | |
| | Benzène + C7⁺ | | | | 0 | 2044 | | | | | | | | 2044 |
| | Coke | | | | | 25 | | | | | | | | |
| | TOTAL | 10000 | 155 | 10155 | 25023 | 26230 | 377 | 1509 | 4067 | 3032 | 12127 | 610 | 2440 | 2044 |

### Exemple 3 (invention) : procédé de conversion directe d'une charge comprenant des oléfines en C4 pour la production de propylène comprenant une étape d'oligocraquage et une étape d'oligomérisation de l'éthylène.

Cet exemple est illustré par la figure 1. La charge à traiter (1) du procédé est une coupe C4 brute issue d'une unité de vapocraquage. Elle présente une composition identique à celle de l'exemple 1 et est donnée dans le tableau 3. L'unité (a) mettant en oeuvre l'étape d'hydrogénation sélective est identique à celle de l'exemple 1. Elle fait appel à l'emploi des 2 réacteurs décrits dans l'exemple 1.

L'unité (b) mettant en oeuvre l'étape d'oligocraquage opère dans les mêmes conditions que celles de l'exemple 1, à la différence que le débit de la charge (2a) qui entre dans ladite unité (b) est supérieur puisqu'il comprend en plus les oligomères formés dans l'unité (d) d'oligomérisation de l'éthylène. L'effluent (4) riche en éthylène recueilli à l'issue de la zone de séparation (c) est envoyé à l'unité (d) mettant en oeuvre ladite étape d'oligomérisation de l'éthylène. Le ratio massique propylène/isobutène dans l'effluent de l'étape d'oligocraquage est égal à 1,5. Le ratio massique propylène/(isobutène + iso-oléfines en C5) dans l'effluent de l'étape d'oligocraquage est égal à 1,08.

L'oligomérisation de l'éthylène est réalisée dans un réacteur de type parfaitement agité, en phase liquide, avec recycle d'une partie de l'effluent dans le réacteur après refroidissement. Le recycle a un rôle de diluant thermique car la réaction est exothermique. La réaction se fait en catalyse homogène en présence d'un catalyseur constitué d'un sel de nickel bivalent dilué dans un acide organique de Bronsted (LC 1252, Axens) et de dichloroéthylaluminium (fourni par la société Crompton) avec un rapport molaire Al / Ni égal à 15/1. Le recycle a un rôle de diluant thermique car la réaction est exothermique. Le réacteur fonctionne à une pression suffisante pour maintenir le système en phase liquide ou au moins suffisante pour ne pas vaporiser trop d'éthylène et à une température comprise typiquement entre 45°C et 55°C. L'effluent du réacteur qui contient du catalyseur est ensuite mélangé avec de l'ammoniac dans une cuve agitée puis le catalyseur est séparé de l'effluent du réacteur par mélange avec une solution aqueuse de soude. La phase aqueuse de soude contenant le catalyseur neutralisé et l'effluent du réacteur (hydrocarbures) sont séparés dans un ballon.

La composition et le débit total des différents flux figurant sur la figure 1 sont indiqués dans le tableau 3.

Le rendement global de la coupe C3 est de 41,3 % poids par rapport à la charge fraîche ((1)+(1a)) du procédé global. Cette coupe C3 contient 93% poids de propylène. L'augmentation du rendement en propylène est donc de 3,4% par rapport à l'exemple 1. Le ratio massique propylène/éthylène sortant du procédé est égal à 434.

**Tableau 3 : Bilan matière (débits en kg/h)**

| | | 1 | 1a | 2 | 2a | 3 | 4 | 5 | 6 | 7 | 8 | 9 | 10 | 11 | 12 | 13 |
|---|---|---|---|---|---|---|---|---|---|---|---|---|---|---|---|---|
| | H2 + methane + ethane | | 155 | | | 88 | 88 | | | | | | | 88 | 88 | |
| | Ethylène | | | | | 903 | 903 | | | | | | | 9 | 9 | |
| C3 | Propylène + propane | | | | | 4197 | | 4197 | | | | | | | | |
| C4 | n oléfines | 2340 | | 6256 | 10614 | 5000 | | | 1000 | 4000 | | | | 359 | | 359 |
| | iso oléfines | 2889 | | 2889 | 4946 | 2571 | | | 514 | 2057 | | | | | | |
| | dioléfines | 3976 | | 2 | 9 | 9 | | | 2 | 7 | | | | | | |
| | paraffines | 795 | | 1008 | 7330 | 7902 | | | 1580 | 6322 | | | | | | |
| C5 | n + iso oléfines | | | | 1240 | 1550 | | | | | 310 | 1240 | | | | |
| | cyclo-oléfines | | | | 31 | 38 | | | | | 8 | 31 | | | | |
| | dioléfines | | | | 0 | 0 | | | | | 0 | 0 | | | | |
| | paraffines | | | | 701 | 876 | | | | | 175 | 701 | | | | |
| C6 | oléfines | | | | 552 | 252 | | | | | 50 | 201 | | 351 | | 351 |
| | paraffines | | | | 345 | 431 | | | | | 86 | 345 | | | | |
| | Benzène + C7⁺ | | | | 185 | 2109 | | | | | | | 2109 | 185 | | 185 |
| | Coke | | | | | 26 | | | | | | | | | | |
| | TOTAL | 10000 | 155 | 10155 | 25952 | 25952 | 991 | 4197 | 3096 | 12385 | 629 | 2518 | 2109 | 991 | 97 | 894 |

La comparaison des résultats figurant dans les tableaux 1, 2 et 3 démontrent que le procédé selon l'invention qui intègre une étape d'oligomérisation de l'éthylène en aval de l'étape d'oligocraquage et le recyclage de l'oligomérat à l'entrée du réacteur d'oligocraquage conduit à un rendement en propylène amélioré par rapport aux procédés antérieurs dans lesquels l'étape d'oligomérisation est inexistante. De plus, le procédé selon l'invention conduit à une augmentation notable du rapport massique propylène/éthylène, signifiant une sélectivité accrue du procédé selon l'invention.

## Revendications

1. Procédé de conversion directe d'une charge comprenant des oléfines à quatre et/ou cinq atomes de carbone pour la production de propylène **caractérisé en ce qu'**il comporte successivement au moins :
1) une étape d'oligocraquage des oléfines présentes dans ladite charge conduisant à la production d'au moins un effluent comprenant de l'éthylène, du propylène et des oléfines ayant de 4 à 6 atomes de carbone,
2) une étape de séparation dudit effluent dans au moins une zone de séparation de manière à produire au moins un effluent riche en éthylène, au moins un effluent riche en propylène et au moins un effluent comprenant des oléfines ayant de 4 à 6 atomes de carbone,
3) une étape d'oligomérisation de l'éthylène produit dans l'étape 1) et recueilli à l'issue de ladite étape 2) produisant un oligomérat majoritairement formé de mono-oléfines linéaires en C4 et C6, ladite étape étant mise en oeuvre par catalyse homogène en présence d'un catalyseur comprenant au moins un composé de nickel bivalent, au moins un halogénure d'hydrocarbyl-aluminium et au moins un acide organique de Brönsted,
4) le recyclage dudit oligomérat produit à ladite étape 3) à l'entrée de l'unité réalisant la mise en oeuvre de ladite étape d'oligocraquage 1).

2. Procédé de conversion selon la revendication 1 tel que ladite charge est choisie parmi les coupes C4 et C5 de l'effluent du FCC et les coupes C4 et C5 brutes de l'effluent du vapocraquage.

3. Procédé de conversion selon la revendication 1 ou la revendication 2 tel qu'une étape d'hydrogénation sélective est mise en oeuvre en amont de ladite étape d'oligocraquage.

4. Procédé de conversion selon la revendication 3 tel que ladite charge comprend une teneur en composés diéniques et acétyléniques supérieures à 100 ppm.

5. Procédé de conversion selon la revendication 3 ou la revendication 4 tel que le catalyseur utilisé pour la mise en oeuvre de ladite étape d'hydrogénation sélective est formé d'au moins un métal du groupe VIII déposé sur au moins un support d'oxyde réfractaire non-acide.

6. Procédé de conversion selon l'une des revendications 1 à 5 tel que ladite étape d'oligocraquage est opérée en une étape.

7. Procédé de conversion selon l'une des revendications 1 à 6 tel que la charge entrant dans l'unité mettant en oeuvre l'étape d'oligocraquage présente une concentration en oléfines tel que la pression partielle en oléfines est comprise entre 0,03 et 0,09 MPa.

8. Procédé de conversion selon l'une des revendications 1 à 7 tel que ledit catalyseur utilisé dans l'unité pour la mise en oeuvre de ladite étape d'oligocraquage comprend au moins une zéolithe présentant une sélectivité de forme choisie parmi les zéolithes présentant un type structural figurant dans la liste suivante : MEL, MFI, NES, EUO, FER, CHA, MFS, MWW ou parmi les zéolithes NU-85, NU-86, NU-88 et IM-5.

9. Procédé de conversion selon l'une des revendications 1 à 8 tel que l'effluent produit par l'étape d'oligocraquage comprend de l'hydrogène, du méthane ainsi que des hydrocarbures dont le point d'ébullition est supérieur à 75°C.

10. Procédé de conversion selon l'une des revendications 1 à 9 tel que ladite zone de séparation opère par distillation.

11. Procédé de conversion selon l'une des revendications 1 à 10 tel que les mono-oléfines linéaires en C4 et C6 présentes dans l'oligomérat à l'issue de ladite étape 3) représentent de 60 à 95% poids des oligomères formés dans l'unité mettant en oeuvre ladite étape d'oligomérisation.

12. Procédé de conversion selon l'une des revendications 1 à 11 tel que ledit catalyseur utilisé pour la mise en oeuvre de ladite étape d'oligomérisation 3) contient au moins un anhydride d'acide carboxylique.

13. Procédé de conversion selon l'une des revendications 1 à 12 tel que l'effluent issu de ladite étape d'oligomérisation 3) est envoyé dans une zone de séparation permettant de recueillir séparément au moins un effluent riche en éthylène n'ayant pas été converti et au moins un effluent comprenant les oligomérats formés au cours de ladite étape d'oligomérisation 3), ledit effluent comprenant lesdits oligomérats étant seul recyclé à l'entrée de l'unité réalisant la mise en oeuvre de ladite étape d'oligocraquage 1).
